# EUROPEAN PATENT SPECIFICATION

(11) **EP 0 597 838 B1**
(45) Date of publication and mention of the grant of the patent: **02.12.1998**
(21) Application number: 91908598.5
(22) Date of filing: 01.05.1991
(51) Int. Cl.: A61K 39/39, A61K 39/29, A61K 39/21

(54) **VACCINE COMPOSITIONS**
IMPFSTOFFZUSAMMENSETZUNGEN
COMPOSITIONS DE VACCINATION

(30) Priority: 07.05.1990 US 518460
(43) Date of publication of application: 25.05.1994
(73) Proprietor: NORTH AMERICAN VACCINE, INC., Beltsville, MD 20705 (CA)
(72) Inventor: PENNY, Christopher, L., Dollard des Ormeaux, PQ, H9A 2S5 (CA)
(74) Representative: Phélip, Bruno
(86) International application number: CA9100144
(87) International publication number: WO9116926

(56) References cited:
- EP-A- 0 018 189
- EP-A- 0 177 015
- EP-A- 0 336 736
- EP-A- 0 356 340
- JOURNAL OF IMMUNOLOGY, vol. 144, no. 12, 15 June 1990, American Association of Immunologists, US; A. NIXON-GEORGE et al., pp. 4798-4802/
- CHEMICAL ABSTRACTS, vol. 103, no. 2, 15 July 1985, Columbus, OH (US); C. PENNEY et al., p. 341, no. 128859z/
- CHEMICAL ABSTRACTS, vol. 105, no. 5, 04 August 1986, Columbus, OH (US); S. LANDI et al., p. 554, no. 40693w/
- CHEMICAL ABSTRACTS, vol. 107, no. 20, 16 November 1987, Columbus, OH (US); C.L. PENNEY et al., p. 503, no. 183681t/
- JOURNAL OF ORGANIC CHEMISTRY, vol. 50, 1985, American Chemical Society; C.L. PENNEY et al., pp. 1457-1459/
- Biologicals, vol. 20, no. 2, June 1992, p. 109-115
- Journal of Bacteriology, vol. 94, no. 3, 1967, p. 586-589
- Annals of the New York Academy of Sciences, vol. 754, 1995, p. 153-160

## Description

The present invention relates to vaccine compositions having improved immunogenicity. More particularly, the compositions of the invention comprise a polypeptide immunogen in combination with a long chain alkyl compound which functions as an immunoadjuvant.

### BACKGROUND OF THE INVENTION

It is well known that vaccines are important in the prophylaxis of disease. Vaccines operate by exposing a host animal to foreign material designed to activate the immune system to confer upon the host immunity against the material without exposing the host to the risk of disease. At the present time, about 20 vaccines have been developed for commercial use. Most of those vaccines are made by detoxification of the disease-causing organism, or a portion of that organism, by one of several methods such as, for example, isolation of a specific non-toxic portion of the organism or by treatment of a toxic portion of the organism with chemicals, heat or genetic attenuation. The detoxification procedure often produces heterogenous products contaminated with toxic by-products of uncertain composition. As a result, the effectiveness of the impure detoxified vaccines as immunogenic materials may be impaired. In addition, unpleasant and sometimes permanently damaging side effects can result. A well known example is the use of intact B. pertussis bacteria as the basis for a vaccine for childhood whooping cough. The vaccine currently used in the United States is essentially the same version introduced in the 1940's. Side effects vary from minor reactions to claimed instances of paralysis, permanent brain damage and even death. European patent application EP-0 336 736 refers to methods of purifying and detoxifying pertussis toxin.

The advent of recombinant and other sophisticated chemical technologies has facilitated the development of purer and hence safer vaccines. However, because of this improved purity, which frequently means that natural immunostimulants have been removed, these new vaccines are often weak, or poorly immunogenic, especially when compared to their predecessors. In order to offset that effect, immunoadjuvants are employed in association with the vaccines to elicit enhanced antibody formation.

Presently, only aluminum and calcium salts are employed as adjuvants for commercial vaccines. However, aluminum and calcium salts are not potent immunoadjuvants. Calcium salts have found only limited use and aluminum salts may provoke transient or chronic local granulomas at the site of injection. L.H.Collier, in Lancet at pages 1364-1367 (1970), states that the incidence and severity of reactions to tetanus toxoid vaccine depends upon the purity of the antigen as well as the presence of aluminum adjuvant. The preparation of aluminum adjuvants is not always reproducible. Moreover, aluminum can alone stimulate the production of IgE antibodies which are responsible for mediating immediate hypersensitive reactions. This has been described by T. Matuhasi, et al. J. Infectious Disease, 146, 290 (1982). European patent application EP 0 177 015 refers to Hepatitis B antigens adjuvanted with aluminum based adjuvants.

Attention has focused in recent years on the use of organic compounds as adjuvants. Only a few organic compounds function in a manner similar to commercially acceptable inorganic salts, i.e. as slow release vehicle or antigen (vaccine) depot whereby antigen is released over a relatively long period of time at the site of injection. Examples of such organic compounds are organic surfactants and emulsifiers, such as Pluronics and Tetronics which are non-ionic block copolymers of polyoxyethylene and polyoxypropylene produced by BASF Corporation. Such a slow-release mechanism of adjuvanticity has long been accepted for human use since it reduces the possibility of overstimulating the immune system. Overstimulation of the immune system can lead to an autoimmune response such as would occur with the use of a potent immunostimulant, for example Freund's adjuvant. Thus, the slow-release mechanism is the preferred mechanism.

While the majority of organic adjuvants have been shown to be potent immunostimulants, such highly active adjuvants tend to be toxic and, therefore, unacceptable for human use. Examples of known organic adjuvants are Freund's complete adjuvant and muramyl dipeptide which are potent immunostimulants, but both of those compounds are restricted to use in animal research because of toxicity considerations. Many of the organic adjuvants which mimic aluminum salts are more toxic than aluminum salts. For example, long chain alkyl amines described by D. Gall in Immunology, 11, 369-386, 1966 are reported to be toxic compounds which are generally disruptive to cell membrane structure.

It is known that large high molecular weight heterogenous antigens of diverse composition will complex with insoluble organic molecules the latter of which function as adjuvants. This is a non-specific binding phenomenon in that, if the antigenic material is large and diverse enough, it will bind to any insoluble organic molecule, regardless of charge and polarity The complexation between the organic adjuvant and the biomolecular occurs through a variety of weak, non-covalent forces, such as hydrophobic interactions and hydrogen bonding.

This phenomenon is seen in U.S. patent 4 428 932 to Overell and U.S patent 4 258 029 to Moloney et al. Overell discloses that alkyl tyrosine functions as an adjuvant for allergy desensitization therapy when complexed with heterogenous multi-component high molecular weight allergens, such as rye, grass and pollen extract. Moloney et al teach the use of long chain alcohol esters of amino acids as adjuvants when complexed with heterogenous multi-component high molecular weight tetanus toxoid and type I,II and III poliomyelitis virus.

Penney et al. (J. Biol. Stand. 13:43-52 (1985)) refers to binding studies of various antigens with different adjuvants, including octadecyl tyrosine. Such antigens include proteins such as tetanus toxoid, diphtheria, toxoid, insulin, and human and bovine albumin. Penney et al. provides no data concerning the immunogenicity of immunogenic compositions comprising purified polypeptides and the various adjuvants suitable for use in the claimed invention.

Landi et al. (Vaccine 4:99-104 (1986)) refers to formalin inactivated poliovirus adjuvanted with stearyl tyrosine hydrochloride and not a homogeneous polypeptide or an essentially pure amino acid sequence . Penney et al. (J. Biol. Stand.14:345-9 (1987)) refers to methods of analysis of stearyl tyrosine. Penney et al. (J. Org. Chem. 50:1457-1459 (1985)) refers to a method for synthesizing long-chain alkyl esters of amino acids and provides no immunological data.

A need exists therefore to develop non-toxic vaccine-adjuvant compositions having improved immunogenicity and in which both the immunogen and the adjuvant are non-toxic.

### SUMMARY OF THE INVENTION

The present invention centers on improved vaccine compositions comprising a homogeneous immunogenic polypeptide and a non-toxic long chain alkyl immunoadjuvant. Thus, according to one aspect of the present invention, there is provided a vaccine composition comprising an essentially pure, as determined by gel electrophoresis or HPLC analysis, immunogenic amino acid sequence and an adjuvant which is a non-toxic long chain alkyl compound present in an amount effective to amplify the immunogenicity of the polypeptide.

According to another aspect of the invention, there is provided a composition for use in a method of eliciting an immune response comprising administering to a warm blooded animal, including humans, an effective amount of a vaccine composition of the invention.

The object of the present invention is specified in claims 1-28.

It has surprisingly been found, according to the present invention, that non-toxic positively charged long chain alkyl compounds, in particular esters of amino acids or peptides, can bind homogeneous polypeptides in a selective manner. This is a specific phenomenon that depends upon the type of amino acid present in the insoluble ester as well as the type of homogeneous polypeptide. This unexpected specificity is characteristic of the homogeneous polypeptides employed according to the invention, and is not exhibited by polysaccharides of similar molecular weight. It has also been found according to the present invention that when the standard aluminum-containing adjuvant is replaced by a non-toxic long chain alkyl compound as adjuvant, typically a long chain alkyl amino acid ester, one or more effects regarding the isotype of antibody produced may possibly occur. One effect concerns IgE antibody production. Specifically, when the long chain alkyl adjuvanted compositions of the invention are used, levels of IgE antibody are not substantially raised and remain essentially at levels observed when no adjuvant is used, as compared to the elevated IgE level observed when aluminum-containing adjuvants are used. IgE antibodies are responsible for mediating immediate hypersensitivity reactions. There have been reports of anaphylatic reactions and even death as a result of IgE production in response to immunization with aluminum adjuvanted vaccines. See Matuhasi, et al, discussed above. Therefore, the reduced levels of IgE formation observed with the long chain alkyl adjuvanted compositions of the invention is a beneficial therapeutic effect.

Another effect is that the ratio of IgG2a antibody to IgG1 is higher in that both antibody isotype levels increased when the long chain alkyl adjuvants are used in the compositions of the invention as opposed to the ratio observed when aluminum adjuvants are used. This ratio increase is also a beneficial effect since IgG2a antibody is important as being the most effective murine antibody in regard to activation of complement and antibody-dependent cellular cytotoxicity mechanisms and protection against tumors and parasites.

The term "homogeneous" as used herein to define polypeptides employed in the vaccine compositions of the invention means an immunogenic polypeptide species consisting essentially of one or more epitopes of a pathogenic agent prepared by chemical synthesis or biological synthesis. In the event that more than one epitope is present, then the homogeneous immunogenic polypeptide would comprise the immunodominant epitope or epitopes. Chemical synthesis is generally employed when one epitope is present, whereas either chemical or biological synthesis may be used in the situation where more than one epitope is present. The homogeneous polypeptide may comprise other portions of the pathogenic agent which are non-toxic and do not adversely affect the immunogenicity of the homogeneous polypeptide.

The non-toxic adjuvant is preferably a positively charged ester of an amino acid or peptide, in particular an ester of an alkyl alcohol containing 14 to 20 carbon atoms and an amino acid, dipeptide, or tripeptide.

### DETAILED DESCRIPTION OF THE INVENTION

The homogeneous immunogenic polypeptide employed in the compositions of the invention is non-toxic and is capable of eliciting an immune response in the host. The immunogenic polypeptide is comprised substantially of one or more epitopes derived from pathogenic agents responsible for AIDS, hepatitis A, hepatitis B, hepatitis C, tetanus, polio, pertussis (Lymphocytosis Promoting Factor (LPF) toxin), herpes simplex, respiratory syncytial virus, measles, influenza virus, rabies, lassa fever, rotavirus, adenovirus, rhinovirus, foot and mouth disease, bovine and feline leukemia virus, rinderpest virus, dengue fever virus, tick-borne encephalitis, malaria and parainfluenza virus.

In many instances, it is desirable to modify the naturally occurring amino acid sequence of the polypeptide immunogen in order to make the homogeneous polypeptide more useful in vaccine compositions, while not changing its immunogenic properties. Examples of such changes include modifications of the amino acid side chains in order to mimic post-translational modifications or to alter the chemical properties of the immunogen. Modifications of the polypeptide in order to improve the lifetime of the immunogen in vivo are also possible. Moreover, addition of a cysteine residue at the amino or carboxyl terminus of the polypeptide in order to facilitate coupling of the polypeptide to a carrier protein is possible.

These changes or modifications may be insertions, deletions and substitutions, either conservative or non-conservative. Such changes include combinations of the naturally occurring amino acids used for protein synthesis, such as gly, ala; val, ile, leu; asp, glu; asn, gln; ser, thr; lys, arg; phe, tyr; ala, ser; ala thr; ala, val; ala, pro; ala, glu; leu, gln; gly, phe; ile, ser; and ile, met.

The immunogenic homogeneous polypeptide may be produced by any convenient method. Thus, the polypeptide may be produced by recombinant or synthetic techniques, or combinations thereof. Known recombinant techniques are described in Molecular Cloning, A Laboratory Manual, Cold Spring Harbor Laboratory, Cold Spring Harbor, New York (1982), T. Maniatis et al. For example, a cell may be designed to overexpress a desired polypeptide through transfection with a gene which codes for that polypeptide.

Suitable chemical synthesis methods include solid- or solution-phase peptide synthesis or enzymatic synthesis. Suitable procedures are described in Principles of Peptide Synthesis, Springer Verlag, 1984, M. Bodanszky; Solid Phase Peptide Synthesis, second edition, 1984, Pierce Chemical Company, John Morrow Stuart and Janis Dillaha Young.

The polypeptide may be either truncated, elongated or otherwise modified by known techniques. Moreover, the polypeptide may be either linear or cyclic.

Purification of the immunogenic polypeptide may be carried out using any commonly employed technique. Any chemical or biosynthetic method which destroys or removes other biomolecules and unwanted material, and leaves the desired immunogenic polypeptide unaffected may be used. Preferably, the immunogenic polypeptide is purified by chromatographic techniques. In particular, chromatographic techniques such as size exclusion, ion exchange, affinity or, most preferably, high performance liquid chromatography, or mixtures thereof, may be employed. The purity of the immunogenic polypeptide may be verified by gel electrophoresis.

The immunogenic polypetides should be of sufficient size to be recognized by the host and identified as foreign. An immunogen usually has a molecular weight of at least 10,000 daltons. If the polypeptide is below the critical size the particular host will recognize, the polypeptide must be coupled to a carrier that will render the polypeptide-carrier conjugate immunogenic. Preferable carriers are proteins. Preferred carriers for animal use are bovine serum albumin and Keyhole Limpet Hemocyanin (KLH). Carriers suitable for human use include tetanus toxoid, diphtheria toxoid, acellular pertussis vaccine (LPF), cross-reacting materials (CRM's) which are antigenically similar to bacterial toxins but are non-toxic by means of mutation, preferably CRM₁₉₇, obtained according to Pappenheimer, et al, An Immunological Study of the Diphtheria Toxin Molecule, Immunochemistry 9, 891-906 (1972), and other bacterial protein carriers, for example meningococcal outer membrane protein. Preferably, the carrier protein itself is an immunogen.

The polypeptide may be coupled to the carrier by any convenient method known to the art. While use of a symmetric linker such as an alkyl dialdehyde (e.g. glutaraldehyde) or an alkyl diimidate (e.g. dimethyl suberimidate) is within the scope of the invention, it is preferred to use a heterobifunctional linker. The latter insures that linkage of the polypeptide to the carrier occurs in a defined and reproducible manner, by avoiding side-reactions such as cross-linkage of carrier molecules which would lead to impure heterogeneous preparations. Examples of preferred heterobifunctional linkers include N-succinimidyl 3-(2-pyridyldithio) propionate as described by J. Carlsson et al; BioChem, J. 173, 723-737 (1978), and sulfosuccinimidyl 4-(p-maleimidophenyl)butyrate, as described by J.D. Bangs, et al; J. Cell Biology 103, 255-263 (1986). Most preferred is sulfosuccinimidyl 4-(N-maleimidomethyl )cyclohexane-1-carboxylate, as described by S. Hashida, et al; J. Applied Biochem 6, 56-63 (1984).

Examples of suitable homogeneous polypeptides are those consisting essentially of substantially the same amino acid sequence as a surface antigen of a hepatitis virus, such as the homogeneous polypeptide consisting essentially of substantially the same amino acid sequence as hepatitis B surface antigen. Another example is a homogeneous polypeptide consisting of one or more epitopes of a surface antigen of hepatitis. Further examples are the homogeneous polypeptide consisting essentially of substantially the same amino acid sequence as a surface antigen of the gD subunit of a herpes simplex virus, the homogeneous polypeptide consisting essentially of one or more epitopes of gp120 of a human immunodeficiency virus, the homogeneous polypeptide consisting essentially of one or more epitopes of gp41 of a human immunodeficiency virus, or the homogeneous polypeptide consisting essentially of substantially the same amino acid sequence as Lymphocytosis Promoting Factor (LPF).

The long chain alkyl adjuvant, as well as any compounds which arise from its metabolism in the host, should be non-toxic. It is well known that long chain fatty alcohols are naturally occurring non-toxic substances. As an example, octadecanol is found to be totally non-toxic in humans, as is indicated by an oral LD₅₀ which is greater than 15 g/kg, as found in Gosselin's "Clinical Toxicology of Commercial Products", Fourth Edition, 1976. Octadecyl tyrosine has been found to be non-toxic in animals and the majority of naturally occurring amino acids are non-toxic. C.L. Penney, et al, Vaccine, 4, 99-10, 1986. It would therefore be expected that octadecyl tyrosine and esters of other alcohols and amino acids would not exhibit any toxicity in humans.

The adjuvant should be capable of forming microparticles having a size of between about 150 µm -1mM (mesh 18 - mesh 100, preferably about 250 µm (mesh 60) in an aqueous medium, thereby giving rise to a suspension of uniform consistency. Moreover, the adjuvant microparticles should permit adsorption of the immunogen, thereby allowing the slow release of immunogen into the host.

In a preferred embodiment of the present invention, the adjuvant is a compound of formula: 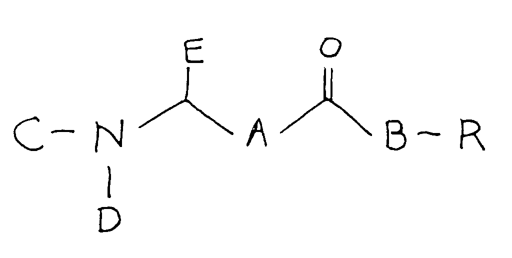 where C is a hydrogen atom, an amino acid residue, or peptide residue including up to ten amino acid residues (i.e. up to a decapeptide); D is a hydrogen atom, or a pharmaceutically acceptable acid such as hydrochloric, hydrobromic, phosphoric, sulfuric, tartaric, lactic or acetic acid; E is 4-hydroxybenzyl, benzyl, 4-hydroxyphenyl, phenyl, 4-aminobutyl, isopropyl, methyl, hydrogen, or other residue of a naturally occurring amino acid; A is (CH₂)ₙ, oxygen or CH₂O and B is (CH₂)ₙ or oxygen, where n is 0 to 4, but A ≠ B for (CH₂)ₙ or oxygen; and R is an alkyl group containing 12 to 20 carbon atoms.

Preferably, C may be either hydrogen, an amino acid, a dipeptide or a tripeptide. If C is an amino acid, the amino acid sequence of the adjuvant may be selected from for example tyrosyl glycine, glycyl glycine, glycyl tyrosine, or phenylalanyl glycine. If C is a dipeptide, the amino acid sequence of the adjuvant may be selected from for example tyrosyl glycyl glycine or tyrosyl alanyl glycine. If an amino acid residue is chiral, the D-enantiomer, the L-enantiomer, or mixtures thereof may be employed. It is particularly preferred for the adjuvant to comprise an alpha amino acid.

It is particularly preferred for E to be selected from 4-hydroxybenzyl, benzyl, 4-hydroxyphenyl, phenyl and hydrogen. E is most preferably 4-hydroxybenzyl.

When A is CH₂O and B is (CH₂)ₙ, the compounds are N-aminoacylethanolamine-O-stearates. When A is CH₂O and B is oxygen, the compounds are carbonates.

More preferably, the adjuvant is an amino acid ester hydrochloride where C is hydrogen, D is hydrochloric acid, A is (CH₂)ₙ, where n is 0-4, and B is oxygen.

Most preferably, the adjuvant is octadecyl tyrosine hydrochloride where C is hydrogen, D is hydrochloric acid, E is 4-hydroxybenzyl, and R is octadecyl, A is (CH₂)ₙ where n is zero and B is oxygen.

Generally, when C is not hydrogen, the backbone of the adjuvant comprises substantially peptide bonds, i.e., the carboxylate of one amino acid residue is linked directly to the amino of the adjacent residue, in a head-to-tail manner. Alternatively, the peptide bond may be a thioamide.

The adjuvant may be prepared by any convenient method. For example, the amino ester portion of the adjuvant may be synthesized by any one of a number of established methods, as has been described by M. Bodanszky et al; "Peptide Synthesis" Second Edition, Wiley, New York, 1976, and R.W. Roeske, Peptides (N.Y.) 3, 102 (1981). A particularly preferred method is the methanesulfonic acid catalyzed esterification procedure described by C. Penney et al, J. Organic Chemistry 50, 1457-1459 (1985).

When the adjuvant is a di- or tripeptide, the peptide bonds may be formed by any of the procedures described in "Peptides Synthesis", mentioned above. Additionally, the peptide bonds may be formed following either solid or solution phase protocols. Many protocols and reagents exist which are useful in forming amide, thioamide, or thioester bonds.

During the preparation of the adjuvant, it may be desirable to temporarily protect reactive functional groups. For example, amines may be protected by urethane-type groups, alcohols by t-butyl or benzyl groups, and acids by ester groups. Suitable protection/deprotection conditions and protocols are described in "Peptide Synthesis" mentioned above.

The adjuvant may be purified by any of the techniques described previously. The preferred purification technique is silica gel chromatography, in particular the "flash" (rapid) chromatographic technique, as described by W. Clark Still et al, J. Organic Chemistry, 43, 2923-2925 (1978). However, other chromatographic methods, including HPLC, may be used for purification of the adjuvant. Crystallization may also be used to purify the adjuvant. In some cases, no purification is required as a product of analytical purity is obtained directly from the synthesis.

The vaccine compositions of the invention are prepared by physically mixing the adjuvant with the homogeneous immunogenic polypeptide under appropriate sterile conditions in accordance with known techniques to produce the adjuvanted composition. As will be clear from the above discussion, the immunogenic polypeptide will be mixed with the adjuvant either on its own or coupled with a suitable carrier.

The amounts of the adjuvant and homogeneous polypeptide immunogen, either alone or coupled to a carrier, needed to elicit an immune response in humans are interrelated, but are within the ranges generally employed in conventional vaccines. For example, the use of increasing amounts of adjuvant may suggest that decreasing amounts of immunogen can be used, and vice versa. The preferred amount of adjuvant is 0.01 to 5 mg/ml of the composition, for example 0.05 mg/ml to 3 mg/ml, preferably 0.5 to 1.0 mg/ml. The preferred amount of immunogen is between about 1 to 100 micrograms/ml, preferably about 5 to 40 micrograms/ml. The dosage will depend upon the host receiving the vaccine as well as factors such as the size, weight, and age of the host.

The vaccine compositions of this invention may be formulated using techniques similar to those used for other pharmaceutical polypeptide compositions. Thus, the adjuvant and immunogen may be stored in lyophilized form and reconstituted in a physiologically acceptable vehicle to form a suspension prior to administration. Alternatively, the adjuvant and immunogen may be stored in the vehicle. Preferred vehicles are sterile solutions, in particular, sterile buffer solutions, such as phosphate buffered saline. Any method of combining the adjuvant and the immunogen in the vehicle that retains the immunoreactivity of the mixture is appropriate.

The vehicle may contain preservatives or other known additives which are used to improve the shelf stability or the efficacy of the mixture. Suitable preservatives include, for example, thimerosal.

The volume of a single dose of the vaccine of this invention may vary but will be generally within the ranges commonly employed in conventional vaccines. The volume of a single dose is preferably between about 0.1 ml and about 1.5 ml, more preferably between about 0.2 ml and about 0.5 ml at the concentrations of immunogen and adjuvant noted above.

The vaccine compositions of the invention may be administered by any convenient means. Preferred methods of administration include subcutaneous, intramuscular, intradermal, or by way of nasal delivery. Alternatively, the mixture may be released from a biodiffusible implant. A single administration may be used. Alternatively, a series of administrations may be made over the course of several days or weeks.

### EXAMPLES

The following non-limiting examples illustrate the invention.

### Example 1

This example describes the preparation of homogeneous Hepatitis B surface antigen (HBsAg). Recombinant HBsAg was isolated from human 3T3 cells which were transfected with the surface antigen of the Hepatitis B virus provided by the Mount Sinai School of Medicine (Sells, et al, Production of Hepatitis B virus particles in HepG2 cells transfected with cloned Hepatitis B virus DNA. Proc. Natl. Acad. Sci. USA, 84,1005 (1987)). Cells were grown in RPMI media (Gibco) supplemented with 10% FCS (Gibco), 1mM sodium pyruvate and 0.5% gentamycin. At this point, two purification procedures may be employed, namely density gradient centrifugation or immunoaffinity chromatography. In density gradient centrifugation, the cell supernatant is spun at 2000 rpm for 10 minutes to remove any 3T3 whole cells, and then the antigen is purified by precipitation using using polyethylene glycol (MW 8000) at a concentration of 10% (w/v). The resulting pellet is resuspended in TNE buffer (10 mM Tris. HCl (pH 8.0), 100 mM NaCl 1 mM EDTA (pH 8.0)) and centrifugation is performed in a gradient of 20% to 50% (wt/vol) cesium chloride in TNE buffer in SW41 polyallomer tubes. The gradients are centrifuged at 35,000 rpm for 60 hours at 4°C. Fractions are collected from the bottom of the tube and the density is determined using a refractometer. The fractions of a density (1.351 - 1.358) are pooled and dialysed against PBS, concentrated and analyzed for homogeneous HBsAg concentration using an Abbot RIA kit using the positive control as equivalent to 20 ng/ml of antigen.

The immunoaffinity chromatography method is generally carried out by spinning the cell supernatant at 2,000 rpm for 10 mins to remove any 3T3 whole cells. An anti-HBsAg affinity column, prepared by covalently linking anti-HBsAg monoclonal antibody to cyanogen bromide activated Sepharose CL4B, is washed with PBS. The OD₂₈₀ nm of eluant is checked, and should be < 0.010. (OD₂₈₀ nm = Optical Density (Absorbence) at 280 nm). The supernatant is run through the column twice to ensure that the antigen is absorbed onto the column. The column is then washed 3 times to remove any non-specific binding of proteins to the column (check O.D.₂₈₀ nm < 0.01), followed by elution with glycine/HCl (pH 2.8). Ten tubes are prepared with sufficient tris buffer (pH 11) in each tube to neutralize the 2ml fraction of glycine/HCl. The ten tubes are analysed by O.D₂₈₀ nm or by measuring HBsAg by RIA. Fractions containing the homogeneous polypeptide are combined and dialyzed against PBS. The column is washed 3 times with PBS. The column is stored in PBS/Na azide.

### Example 2

The following describes the general method for the complexation of a homogeneous polypeptide immunogen with a long chain alkyl amino acid ester immunoadjuvant.

The long chain alkyl amino acid ester immunoadjuvant (50mg) was placed in a 25 ml volumetric or small erlenmeyer flask with spin-bar, and stoppered with a cotton plug, or other loose fitting material. The salt form of the immunoadjuvant was sterilized by heating in an oven at an appropriate temperature, as dictated by the melting point, for 20 hours. To the cooled flask was added phosphate buffered saline (25ml), sterilized by filtration, >50 mM, pH = 6-7. The resulting suspension was gently stirred for 3-20 hours, the plug replaced with a glass stopper, and the whole stored at 4°C until required for complexation.

In order to complex the immunoadjuvant with the homogeneous polypeptide, the suspension was brought to room temperature, and thorougly agitated. The required volume of suspension was transferred with a wide-bore pipet to an equal volume of polypeptide solution, so that the final concentration of immunoadjuvant was 1 mg/ml. However, volume adjustments could be made as required to give any other desired concentration of immunoadjuvant. The immunoadjuvant-polypeptide suspension was gently mixed overnight at 4°C. At the end of the complexation reaction, the suspension was allowed to settle, centrifuged, if necessary, and the absorbance at 280 nm of the supernatent measured to determine the amount of unbound polypeptide. Generally, 30%-90% bound polypeptide represents a good immunoadjuvant-polypeptide immunogen complex.

### Example 3

This example demonstrates the adjuvanticity of several octadecyl amino acid esters with a homogeneous polypeptide which is a candidate vaccine for hepatitis.

BALB/c mice were immunized with 50 ng of hepatitis B surface antigen in the presence of adjuvant for the non-control mice and in the absence of adjuvant for the control mice. The hepatitis B surface antigen was obtained from 3T3 cells transfected with the gene for the surface antigen. 100 µg of octadecyl amino acid was complexed with the polypeptide as described in Example 2. This mixture, in a total volume of 0.1 ml, was injected on day 1 using an intramuscular route, and the mice were bled on days 7, 14, 21, 28, 35, 42 and 61. The mice were boosted with 35 ng of the polypeptide on day 21.

The antibody concentration in the sera was determined by radioimmunoassay. The antibody concentration was estimated by comparison with a standard curve obtained using a monoclonal antibody. The results are presented in Table 1.

**TABLE 1**

| Antibody response to hepatitis B surface antigen in the presence of octadecyl amino acid immunoadjuvant | | | | | | |
|---|---|---|---|---|---|---|
| Adjuvant | DAY* | | | | | |
| | 7 | 14 | 21 | 28 | 35 | 61 |
| Saline | 43 | 36 | 32 | 61 | 59 | 58 |
| Alum | 44 | 53 | 46 | 163 | 320 | 516 |
| Octadecyl Tyrosine | 68 | 91 | 58 | 355 | 833 | 752 |
| Octadecyl β-Alanine | 31 | 45 | 44 | 120 | 337 | 373 |
| Octadecyl Glycine | 47 | 38 | 51 | 147 | 748 | 301 |
| Octadecyl Proline | 49 | 35 | 41 | 72 | 409 | 168 |
| Octadecyl Leucine | 48 | 42 | 37 | 160 | 590 | 496 |
| Octadecyl Lysine | 33 | 55 | 39 | 51 | 67 | 53 |
| Octadecyl Forphenicinol | 31 | 40 | 26 | 65 | 306 | 98 |
| Octadecyl Forphenicine | 100 | 112 | 116 | 908 | 592 | 355 |

| | | | | | | |
|---|---|---|---|---|---|---|
| *Anti-HBsAg antibody concentration, ng/ml | | | | | | |

The hepatitis B surface antigen amino acid sequence for amino acids 1 through 226 (HBsAg - adw (S gene product) is described in Bhatnagar P.K., et al, Proc. Natl. Aca. Science, USA vol. 79, 4400-4404 (1982), specifically incorporated by reference.

The results in Table 1 show that the adjuvant effect observed between long chain alkyl amino acid esters and an immunogenic polypeptide, hepatitis B surface antigen, is a specific phenomenon. This can be seen by comparison of octadecyl tyrosine and octadecyl lysine, and by comparison of octadecyl forphenicinol and octadecyl forphenicine.

### Example 4

This example demonstrates the adjuvanticity of octadecyl tyrosine as a function of dose with a polypeptide which is a candidate vaccine for hepatitis.

The protocol was as described above, except that mice were immunized with 80 ng of hepatitis B surface antigen, boosted with 50 ng, and bled on days 7, 14, 28 and 35. The immunizations were carried out in the presence of adjuvant for the non-control mice and in the absence of adjuvant for the control mice. The results are presented in Table 2.

**TABLE 2**

| Antibody response to hepatitis B surface antigen in the presence of octadecyl tyrosine | | | | | | |
|---|---|---|---|---|---|---|
| Adjuvant | % Adsorbed Antigen | **DAY******* | | | | |
| | | 7 | 14 | 21 | 28 | 35 |
| Saline | 0 | 188 | 40 | 13 | 2388 | 702 |
| Alum | 73 | 880 | 761 | 639 | 12310 | 8726 |
| 100 µg | 70 | 1107 | 537 | 1279 | 19679 | 13427 |
| 50 µg | 60 | 2064 | 846 | 4000 | 18804 | 20616 |
| 10 µg | 53 | 611 | 2314 | 342 | 8403 | 10005 |

| | | | | | | |
|---|---|---|---|---|---|---|
| *CPM, pre-bleed value was 11.5 | | | | | | |

The results in Table 2 show that the adjuvant effect is dependent upon the concentration of long chain alkyl ester, octadecyl tyrosine, present in the composition. As the amount of adjuvant administered increases, a proportional increase in the antibody titer occurs until an optimum titer value is reached, beyond which the antibody titer does not increase.

### Example 5

This example demonstrates the adjuvanticity of octadecyl tyrosine as a function of dose, and in different animals, with a polypeptide which corresponds to a fusiogenic sequence from the envelope protein of the human immunodeficiency virus, HIV-1.
1) HIV Peptide Sequence
The peptide sequence of the HIV was as follows.
Product: BC-101
503-
H-Val-Ala-Pro-Thr-Lys-Ala-Lys-Arg-Arg-Val-Val-Gln-Arg-Glu-Lys-Arg-Ala-Val-Gly-Ile-Gly-Ala-Leu-Phe-Leu-Gly-Phe-Leu-Gly-Ala-Ala-Gly-Ser- 535 M.W. 3585.14 (Back-bone)
2) Experiments in mice
BALB/c mice were immunized with 100 µg of chemically synthesized polypeptide, corresponding to the amino acid sequence of residues 503-535 of HIV-1, coupled to Keyhole Limpet Hemocyanin (KLH). Octadecyl tyrosine hydro-chloride, 10 µg-100 µg, was complexed with the polypeptide as described in Example 2. This mixture, in a total volume of 0.1 ml, was injected on day 1, and again on day 21. The mice were bled on days 7, 14, 21, 28 and 35.
The antibody concentration in the sera was determined by radioimmunoassay. The antibody concentration was estimated by comparison with a standard curve obtained using a polyclonal antibody. The results are presented in Table 3.

**TABLE 3**

| Antibody response to HIV-1 polypeptide, residues 503-535, in the presence of octadecyl tyrosine | | | | | |
|---|---|---|---|---|---|
| Adjuvant | DAY* | | | | |
| | 7 | 14 | 21 | 28 | 35 |
| Saline | 1.04 | 0.84 | 1.03 | 8.56 | 11.19 |
| Alum | 1.59 | 5.54 | 3.16 | 86.6 | 82.1 |
| 100 µg | 0.87 | 1.65 | 2.04 | 155.3 | 138.0 |
| 50 µg | 1.18 | 0.75 | 2.99 | 54.2 | 86.2 |
| 10 µg | 1.61 | 3.15 | 1.49 | 85.9 | 131.9 |

| | | | | | |
|---|---|---|---|---|---|
| *Anti-peptide antibody concentration, µg/ml | | | | | |

The results in Table 3 show that a long chain alkyl ester, octadecyl tyrosine, exhibits a concentration dependent adjuvant effect with another immunogenic polypeptide, namely a polypeptide derived from amino acids 503 to 535 of the HIV protein gp41. Again, an optimum antibody titer is reached, beyond which the titer does not increase.
3) Experiments in baboons
Two baboons were immunized with 300 µg of chemically synthesized polypeptide, corresponding to the amino acid sequence of residues 503-535 of HIV-1, coupled to Keyhole Limpet Hemocyanin. 500 µg of octadecyl tyrosine hydrochloride was complexed with the polypeptide, as described in Example 2. This mixture in a total volume of 1.0 ml was injected on day 1 and again on day 35. The animals were bled 3 days before the first immunization, and on days 14, 32 and 49. The same experiment was undertaken with another two baboons, but with polypeptide corresponding to the amino acid sequence 526-535 of HIV-1.
The antibody concentration in the sera was indirectly measured by radioimmunoassay. Tiltration was undertaken by incubation of samples on plates coated with peptide-albumin conjugate, and reaction with iodine-125 labelled rabbit anti-monkey immunoglobulin. The results are presented in Table 4.

**TABLE 4**

| Antibody response to HIV-1 polypeptide in baboons in the presence of 500 µg octadecyl tyrosine hydrochloride | | | | | | | | |
|---|---|---|---|---|---|---|---|---|
| Polypeptide | ANIMAL 1 | | | | ANIMAL 2 | | | |
| | DAY* | | | | DAY* | | | |
| | -3 | 14 | 32 | 49 | -3 | 14 | 32 | 49 |
| 503-535 | 1020 | 2004 | 2185 | 5948 | 1530 | 2160 | 2121 | 4470 |
| 526-535 | 300 | 686 | 1685 | 3121 | 220 | 500 | 965 | 4373 |

| | | | | | | | | |
|---|---|---|---|---|---|---|---|---|
| *CPM, after subtraction of background on bovine serum albumin coated plates | | | | | | | | |

The results in Table 4 show that the adjuvant effect between a long chain alkyl ester, octadecyl tyrosine, and an immunogenic HIV polypeptide is observed in higher animals, for example baboons.

### Example 6

This example demonstrates the change in isotype which occurs in going from an aluminum adjuvanted to an octadecyl tyrosine adjuvanted candidate vaccine for hepatitis.

The protocol was as described in Example 5, Section 2 (mice) except that 50 µg of octadecyl tyrosine was used for both the primary and secondary (day 21) immunizations. The isotype of the antibody response was determined by radioimmunoassay. Use was made of tritium labelled anti-mouse isotype antibody. Titration was undertaken by incubation of samples on plates coated with immunoaffinity purified hepatitis B surface antigen. The results reported as radioactivity (cpm) of bound isotype antibody, are presented in Table 5.

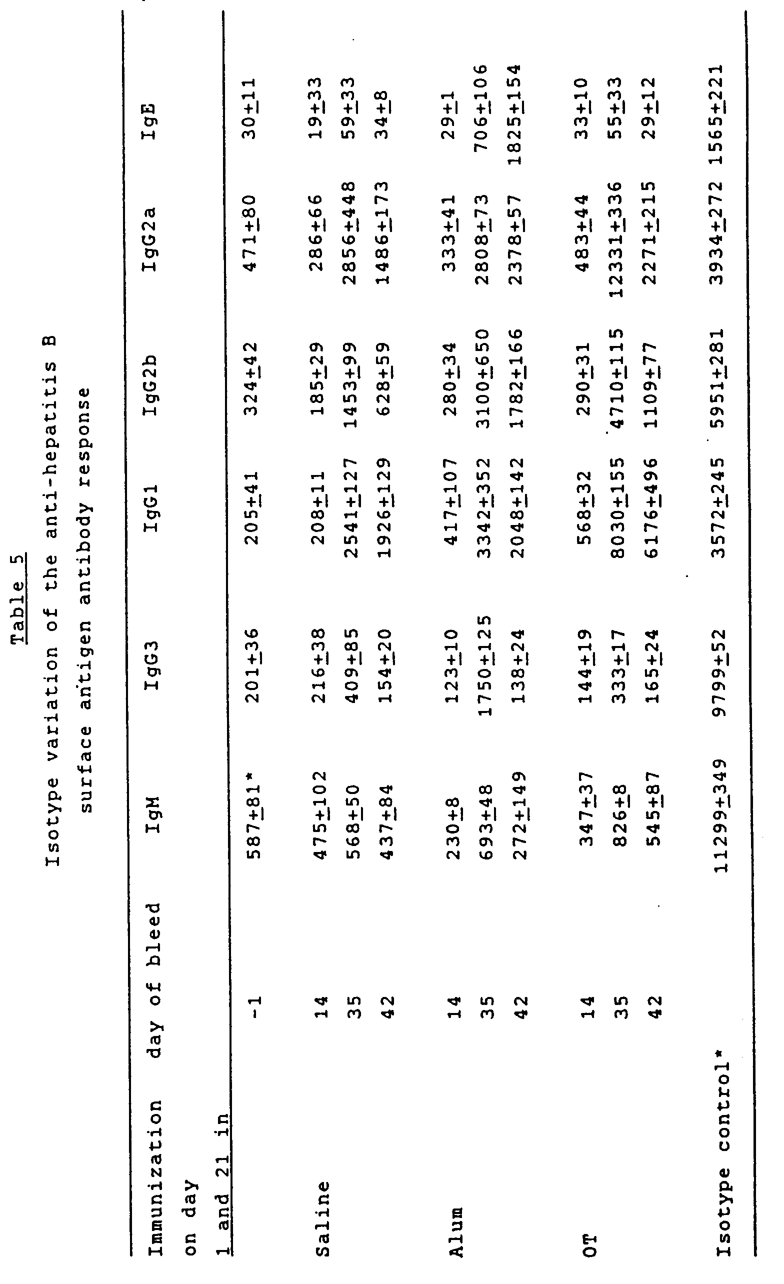

The results shown in table 5 show the change in isotype of the antibody response which occurs in going from aluminum to long chain alkyl ester, (octadecyl tyrosine - OT) adjuvanted immunogenic polypeptide, hepatitis B surface antigen. In particular, it is to be noted that the levels of IgE antibodies are elevated when aluminum is used, whereas there is no significant increase in IgE levels, as compared to that obtained with the control, when octadecyl tyrosine (OT) is used.

## Claims

1. Immunogenic composition comprising an essentially pure, as determined by gel electrophoresis or HPLC analysis, immunogenic amino acid sequence and an effective amount of at least one adjuvant of the formula: 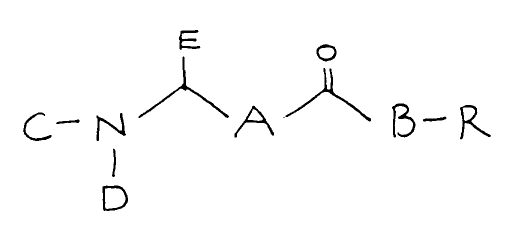
wherein C is selected from the group consisting of hydrogen, an amino acid residue, and a peptide residue;
D is selected from the group consisting of hydrogen and a pharmaceutically acceptable acid;
E is selected from the group consisting of 4-hydroxybenzyl, benzyl, 4-hydroxyphenyl, phenyl, 4-aminobutyl, isopropyl, methyl, hydrogen and a residue of a naturally occurring amino acid;
A is (CH₂)ₙ, oxygen or CH₂O and B is (CH₂)ₙ or oxygen, wherein n is O to 4, with the proviso that A and B are not the same for (CH₂)ₙ and oxygen; and
R is alkyl of 12 to 2O carbon atoms.

2. Composition according to claim 1, wherein said adjuvant comprises an amino acid having an L-configuration.

3. Composition according to claim 1, wherein said adjuvant comprises an amino acid having a D-configuration.

4. Composition according to claim 1, wherein said adjuvant comprises an amino acid mixture of D and L-configurations.

5. Composition according to claim 1, wherein E is selected from the group consisting of 4-hydroxybenzyl, benzyl, 4-hydroxyphenyl, phenyl, and hydrogen.

6. Composition according to claim 5, wherein E is 4-hydroxybenzyl.

7. Composition according to claim 1, wherein C is selected from the group consisting of hydrogen, an amino acid, and a peptide residue including up to ten amino acid residues.

8. Composition according to claim 7, wherein said peptide residue is selected from a dipeptide and a tripeptide.

9. Composition according to claim 7, wherein C is an amino acid selected so as to form an adjuvant comprising a dipeptide selected from the group consisting of tyrosyl glycine, glycyl glycine, glycyl tyrosine, and phenylalanyl glycine.

10. Composition according to claim 7, wherein C is a dipeptide selected so as to form an adjuvant comprising a tripeptide selected from the group consisting of tyrosyl glycyl glycine and tyrosyl alanyl glycine.

11. Composition according to claim 1, wherein the pharmaceutically acceptable acid is selected from the group consisting of hydrochloric, hydrobromic, phosphoric, sulfuric, tartaric, lactic, and acetic acid.

12. Composition according to claim 1, wherein said adjuvant molecule comprises an α-amino acid.

13. Composition according to claim 1, wherein said amino acid sequence is selected from the group consisting of a linear and a cyclic polypeptide.

14. Composition according to claim 1, wherein said essentially pure immunogenic amino acid sequence is coupled to a carrier protein.

15. Composition according to claim 14, wherein said carrier is selected from the group consisting of tetanus toxoid, diphtheria toxoid, acellular pertussis vaccine (LPS), a cross-reacting material (CRM) and a bacterial protein carrier.

16. Composition according to claim 15, wherein said CRM is CRM₁₉₇.

17. Composition according to claim 1, wherein said aminoacid sequence is derived from a pathogenic agent responsible for a disease selected from AIDS, hepatitis A, hepatitis B, hepatitis C, tetanus, polio, pertussis (Lymphocytosis Promoting Factor toxin), herpes simplex, respiratory syncytial virus, measles, influenza virus, rabies, lassa fever, rotavirus, adenovirus, rhinovirus, foot and mouth disease, bovine and feline leukemia virus, rinderpest virus, dengue fever virus, tick-borne encephalitis, malaria and parainfluenza virus.

18. Composition according to claim 1, wherein said amino acid sequence is a surface antigen of a hepatitis virus.

19. Composition according to claim 1, wherein said aminoacid sequence comprises at least one epitope of a surface antigen of a hepatitis virus.

20. Composition according to claim 1, wherein said amino acid sequence is a hepatitis B surface antigen.

21. Composition according to claim 1, wherein said amino acid sequence is a surface antigen of the gD subunit of a herpes simplex virus.

22. Composition according to claim 1, wherein said amino acid sequence comprises at least one epitope of gp120 of a human immunodeficiency virus.

23. Composition according to claim 1, wherein said amino acid sequence comprises at least one epitope of gp41 of a human immunodeficiency virus.

24. Composition according to claim 1, wherein said amino acid sequence is Lymphocytosis Promoting Factor.

25. Composition according to claim 1, wherein said adjuvant is an ester of an alkyl alcohol containing 14 to 20 carbon atoms and an amino acid, dipeptide or tripeptide.

26. Composition according to claim 25, wherein said adjuvant is octadecyl tyrosine.

27. Composition according to one of claims 1 to 26 for intramuscularly, intradermally, subcutaneously, or nasal delivery use.

28. Use of a composition according to one of claims 1 to 27 for the manufacture of a vaccine for eliciting an immune response against a pathogenic agent.

## Patentansprüche

1. Immunogene Zusammensetzung, umfassend eine gemäß Gelelektrophorese oder HPLC-Analyse im wesentlichen reine, immunogene Aminosäuresequenz und eine wirksame Menge wenigstens eines Adjuvans der Formel 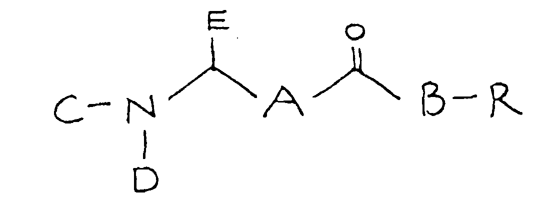
wobei C aus der Gruppe ausgewählt ist, die aus Wasserstoff, einem Aminosäurerest und einem Peptidrest besteht,
D aus der Gruppe ausgewählt ist, die aus Wasserstoff und einer pharmazeutisch annehmbaren Säure besteht,
E aus der Gruppe ausgewählt ist, die aus 4-Hydroxybenzyl, Benzyl, 4-Hydroxyphenyl, Phenyl, 4-Aminobutyl, Isopropyl, Methyl, Wasserstoff und einem Rest einer natürlich vorkommenden Aminosäure besteht,
A (CH₂)ₙ, Sauerstoff oder CH₂O ist und B (CH₂)ₙ oder Sauerstoff ist, wobei n 0 bis 4 ist, mit der Maßgabe, daß A und B für(CH₂)ₙ und Sauerstoff nicht gleich sind, und
R Alkyl mit 12 bis 20 Kohlenstoffatomen ist.

2. Zusammensetzung gemäß Anspruch 1, wobei das Adjuvans eine Aminosäure mit einer L-Konfiguration umfaßt.

3. Zusammensetzung gemäß Anspruch 1, wobei das Adjuvans eine Aminosäure mit einer D-Konfiguration umfaßt.

4. Zusammensetzung gemäß Anspruch 1, wobei das Adjuvans ein Aminosäuregemisch mit D- und L-Konfigurationen umfaßt.

5. Zusammensetzung gemäß Anspruch 1, wobei E aus der Gruppe ausgewählt ist, die aus 4-Hydroxybenzyl, Benzyl, 4-Hydroxyphenyl, Phenyl und Wasserstoff besteht.

6. Zusammensetzung gemäß Anspruch 5, wobei E 4-Hydroxybenzyl ist.

7. Zusammensetzung gemäß Anspruch 1, wobei C aus der Gruppe ausgewählt ist, die aus Wasserstoff, einem Aminosäurerest und einem Peptidrest mit bis zu zehn Aminosäureresten besteht.

8. Zusammensetzung gemäß Anspruch 7, wobei der Peptidrest aus einem Dipeptid und einem Tripeptid ausgewählt ist.

9. Zusammensetzung gemäß Anspruch 1, wobei C ein Aminosäurerest ist, der so ausgewählt ist, daß er ein Adjuvans bildet, das ein Dipeptid umfaßt, das aus der Gruppe ausgewählt ist, die aus Tyrosylglycin, Glycylglycin, Glycyltyrosin und Phenylalanylglycin besteht.

10. Zusammensetzung gemäß Anspruch 7, wobei C ein Dipeptid ist, das so ausgewählt ist, daß es ein Adjuvans bildet, das ein Tripeptid umfaßt, das aus der Gruppe ausgewählt ist, die aus Tyrosylglycylglycin und Tyrosylalanylglycin besteht.

11. Zusammensetzung gemäß Anspruch 1, wobei die pharmazeutisch annehmbare Säure aus der Gruppe ausgewählt ist, die aus Chlorwasserstoff-, Bromwasserstoff-, Phosphor-, Schwefel-, Wein-, Milch- und Essigsäure besteht.

12. Zusammensetzung gemäß Anspruch 1, wobei das Adjuvansmolekül eine α-Aminosäure umfaßt.

13. Zusammensetzung gemäß Anspruch 1, wobei die Aminosäuresequenz aus der Gruppe ausgewählt ist, die aus einem linearen und einem cyclischen Polypeptid besteht.

14. Zusammensetzung gemäß Anspruch 1, wobei die im wesentlichen reine, immunogene Aminosäuresequenz an ein Trägerprotein gekoppelt ist.

15. Zusammensetzung gemäß Anspruch 14, wobei der Träger aus der Gruppe ausgewählt ist, die aus Tetanus-Toxoid, Diphtherie-Toxoid, acellulärem Pertussis-Impfstoff (LPS), einem kreuzreagierenden Material (CRM) und einem bakteriellen Proteinträger besteht.

16. Zusammensetzung gemäß Anspruch 15, wobei es sich bei dem CRM um CRM₁₉₇ handelt.

17. Zusammensetzung gemäß Anspruch 1, wobei die Aminosäuresequenz von einem pathogenen Mittel abgeleitet ist, das für eine Krankheit verantwortlich ist, die aus AIDS, Hepatitis A, Hepatitis B, Hepatitis C, Tetanus, Polio, Pertussis (Lymphocytose-fördernder-Faktor-Toxin), Herpes simplex, respiratorischem syncytialem Virus, Masern, Influenzavirus, Tollwut, Lassafieber, Rotavirus, Adenovirus, Rhinovirus, Maul- und Klauenseuche, Rinder- und KatzenLeukämievirus, Rinderpestvirus, Denguefiebervirus, durch Zecken übertragener Encephalitis, Malaria und Parainfluenzavirus ausgewählt ist.

18. Zusammensetzung gemäß Anspruch 1, wobei die Aminosäuresequenz ein Oberflächenantigen eines Hepatitisvirus ist.

19. Zusammensetzung gemäß Anspruch 1, wobei die Aminosäuresequenz wenigstens ein Epitop eines Oberflächenantigens eines Hepatitisvirus umfaßt.

20. Zusammensetzung gemäß Anspruch 1, wobei die Aminosäuresequenz ein Hepatitis-B-Oberflächenantigen ist.

21. Zusammensetzung gemäß Anspruch 1, wobei die Aminosäuresequenz ein Oberflächenantigen der gD-Untereinheit eines Herpes-simplex-Virus ist.

22. Zusammensetzung gemäß Anspruch 1, wobei die Aminosäuresequenz wenigstens ein Epitop von gp120 eines humanen Immunschwächevirus (HIV) umfaßt.

23. Zusammensetzung gemäß Anspruch 1, wobei die Aminosäuresequenz wenigstens ein Epitop von gp41 eines humanen Immunschwächevirus (HIV) umfaßt.

24. Zusammensetzung gemäß Anspruch 1, wobei es sich bei der Aminosäuresequenz um Lymphocytose-fördernden Faktor handelt.

25. Zusammensetzung gemäß Anspruch 1, wobei das Adjuvans ein Ester eines Alkylalkohols mit 14 bis 20 Kohlenstoffatomen und einer Aminosäure, eines Dipeptids oder eines Tripeptids ist.

26. Zusammensetzung gemäß Anspruch 25, wobei es sich bei dem Adjuvans um Octadecyltyrosin handelt.

27. Zusammensetzung gemäß einem der Ansprüche 1 bis 26 für intramuskuläre, intradermale, subcutane oder nasale Verabreichung.

28. Verwendung einer Zusammensetzung gemäß einem der Ansprüche 1 bis 27 zur Herstellung eines Impfstoffs zum Hervorrufen einer Immunreaktion gegen einen Krankheitserreger.

## Revendications

1. Composition pour l'induction d'une réponse immunitaire comprenant une séquence d'acides aminés pour l'induction d'une réponse immunitaire essentiellement purs comme déterminé par électrophorèse sur gel ou analyse HPLC, et une quantité efficace d'au moins un adjuvant de formule : 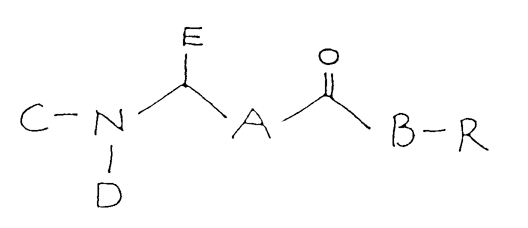
dans laquelle C est choisi parmi le groupe comprenant l'hydrogène, un reste d'acide aminé et un reste peptidique,
D est choisi parmi le groupe comprenant l'hydrogène et un acide acceptable du point de vue pharmaceutique.
E est choisi parmi le groupe comprenant un radial 4-hydroxybenzyle, benzyle, 4-hydroxyphényle, phényle, 4-aminobutyle, isopropyle, méthyle, l'hydrogène et un reste d'acide aminé d'origine naturelle.
A est (CH₂)ₙ, l'oxygène ou CH₂O et B est (CH₂)ₙ ou l'oxygène, où n est compris entre 0 et 4, à condition que A et B ne soient par semblables pour (CH₂)ₙ et l'oxygène, et
R est un radial alkyle de 12 à 20 atomes de carbone.

2. Composition selon la revendication 1, dans laquelle ledit adjuvant comprend un acide aminé ayant une configuration L.

3. Composition selon la revendication 1, dans laquelle ledit adjuvant comprend un acide aminé ayant une configuration D.

4. Composition selon la revendication 1, dans laquelle ledit adjuvant comprend un mélange d'acides aminés de configurations D et L.

5. Composition selon la revendication 1, dans laquelle E est choisi parmi le groupe comprenant un radial 4-hydroxybenzyle, benzyle, 4-hydroxyphényle, phényle et l'hydrogène.

6. Composition selon la revendication 5, dans laquelle E est un radical 4-hydroxybenzyle.

7. Composition selon la revendication 1, dans laquelle C est choisi parmi le groupe comprenant l'hydrogène, un acide aminé, et un reste peptidique renfermant jusqu'à dix restes d'acides aminés.

8. Composition selon la revendication 7, dans laquelle ledit reste de peptide est choisi parmi un dipeptide et un tripeptide.

9. Composition selon la revendication 7, dans laquelle C est un acide aminé choisi de façon à former un adjuvant comprenant un dipeptide choisi parmi le groupe comprenant la tyrosyl-glycine, la glycyl-glycine, la glycyl-tyrosine, et la phénylalanyl-glycine.

10. Composition selon la revendication 7, dans laquelle C est un dipeptide choisi de façon à former un adjuvant comprenant un tripeptide choisi parmi le groupe comprenant la tyrosyl-glycyl-glycine et la tyrosyl-alanyl-glycine.

11. Composition selon la revendication 1, dans laquelle l'acide acceptable du point de vue pharmaceutique est choisi parmi le groupe comprenant l'acide chlorhydrique, l'acide bromhydrique, l'acide phosphorique, l'acide sulfurique, l'acide tartrique, l'acide lactique et l'acide acétique.

12. Composition selon la revendication 1, dans laquelle ladite molécule d'adjuvant renferme un acide α-aminé.

13. Composition selon la revendication 1, dans laquelle ladite séquence d'acides aminés est choisie parmi le groupe comprenant un polypeptide linéaire et un polypeptide cyclique.

14. Composition selon la revendication 1, dans laquelle ladite séquence d'acides aminés pour l'induction d'une réponse immunitaire essentiellement pure est couplée à une protéine de support.

15. Composition selon la revendication 14, dans laquelle ledit support est choisi parmi le groupe comprenant le toxoide du tétanos, le toxoide de la diphtérie, le vaccin de la coqueluche acellulaire (LPS), un matériau de réaction croisée (CRM) et un support de protéine bactérienne.

16. Composition selon la revendication 15, dans laquelle ledit CRM est CRM₁₉₇.

17. Composition selon la revendication 1, dans laquelle ladite séquence d'acides aminés est dérivée d'un agent pathogène responsable d'une maladie choisie parmi le SIDA, l'hépatite A, l'hépatite B, l'hépatite C, le tétanos, la poliomyélite, la coqueluche (toxine du facteur favorisant la lymphocytose), l'herpès simple, le virus syncytial respiratoire, la rougeole, le virus de la grippe, la rage, la fièvre de lassa, le rotavirus, l'adénovirus, le rhinovirus, la maladie de la bouche et du pied, le virus de la leucémie des bovins et des félins, le virus de la peste bovine, le virus de la fièvre de la dengue, l'encéphalite liée aux tiques, la malaria et le virus de la grippe para.

18. Composition selon la revendication 1, dans laquelle ladite séquence d'acides aminés est un antigène de surface d'un virus de l'hépatite.

19. Composition selon la revendication 1, dans laquelle ladite séquence d'acides aminés comprend au moins un épitote d'un antigène de surface d'un virus de l'hépatite.

20. Composition selon la revendication 1, dans laquelle ladite séquence d'acides aminés est un antigène de surface de l'hépatite B.

21. Composition selon la revendication 1, dans laquelle ladite séquence d'acides aminés est un antigène de surface de la sous-unité gD d'un virus de l'herpès simple.

22. Composition selon la revendication 1, dans laquelle ladite séquence d'acides aminés comprend au moins un épitote de gp120 d'un virus d'immunodéficience d'un humain.

23. Composition selon la revendication 1, dans laquelle ladite séquence d'acides aminés comprend au moins un épitote de gp41 d'un virus d'immunodéficience d'un humain.

24. Composition selon la revendication 1, dans laquelle ladite séquence d'acides aminés est un facteur favorisant la lymphocytose.

25. Composition selon la revendication 1, dans laquelle ledit adjuvant est un ester d'un alkyl-alcool renfermant de 14 à 20 atomes de carbone et d'un acide aminé, d'un dipeptide ou d'un tripeptide.

26. Composition selon la revendication 25, dans laquelle ledit adjuvant est l'octadécyl-tyrosine.

27. Composition selon l'une des revendications 1 à 26 pour une utilisation intramusculaire, intradermique, sous-cutanée ou par voie nasale.

28. Utilisation d'un composition selon l'une des revendications de 1 à 27 pour la fabrication d'un vaccin pour l'induction d'une réponse immunitaire contre un agent pathogène.
